# FASCICULE DE BREVET EUROPEEN

(11) **EP 1 196 102 B1**
(45) Date de publication et mention de la délivrance du brevet: **01.09.2004**
(21) Numéro de dépôt: 00946016.3
(22) Date de dépôt: 26.06.2000
(51) Int. Cl.: A61B 17/70

(54) **CONNECTEUR POLYAXIAL POUR IMPLANT RACHIDIEN**
MEHRACHSIGE VERBINDUNGSEINRICHTUNG FÜR WIRBELSÄULENIMPLANTAT
MULTIAXIAL CONNECTOR FOR SPINAL IMPLANT

(30) Priorité: 23.07.1999 FR 9909755
(43) Date de publication de la demande: 17.04.2002
(73) Titulaire: EUROSURGICAL, 62217 Beaurains (FR)
(72) Inventeur: MARTIN BENLLOCH, Antonio, E-46022 Valence (ES); LEROY, Jean-Yves, F-62870 Campagne les Hesdin (FR); PEREZ PEDRON, Inmaculada, Urb. Altos Blancos, 03580 el Campo-Alicante (ES); VIART, Guy, F-62128 Saint Leger (FR)
(74) Mandataire: Schmitt, John
(86) Numéro de dépôt international: PCT/FR2000/001781
(87) Numéro de publication internationale: WO 2001/006939

(56) Documents cités:
- EP-A- 0 468 264
- FR-A- 2 761 590
- FR-A- 2 765 093

## Description

La présente invention est relative à un connecteur polyaxial pour la constitution d'un implant rachidien muni de tiges de liaison.

On connaît d'après le brevet français n° 2 731 344 un implant rachidien permettant l'orientation angulaire d'une tige de liaison par rapport aux éléments d'ancrage osseux.

L'élément d'ancrage est constitué d'une vis de fixation comportant une partie d'ancrage séparée d'une tête filetée saillante, associé à un écrou de serrage, par un corps intermédiaire de section polygonale formant butée.

L'élément d'ancrage comporte également une bague de réception qui coopère avec la tête filetée saillante de la partie d'ancrage et qui permet la mise en place de la tige de liaison.

La bague de réception comporte deux branches réunies par une zone de liaison pour constituer une élément formant pince. Les deux branches sont percées de trous coaxiaux en vis à vis, prévus pour être traversés par la tête filetée saillante de la vis de fixation, et venir en appui contre la butée polygonale.

La zone de liaison des deux branches de la bague délimite perpendiculairement aux trous en vis à vis un alésage coopérant avec une douille compressible muni d'un passage central prévu pour recevoir la tige de liaison.

L'assemblage de la douille à l'intérieur de l'alésage de la bague est destiné à créer une liaison rotule pour pouvoir positionner, avant le serrage de l'écrou sur la tête filetée saillante de la vis de fixation, la tige de liaison dans une position angulaire déterminée.

L'implant rachidien décrit ci-dessus comporte certains inconvénients en ce qui concerne le montage de la douille à l'intérieur de l'alésage de la bague formant la liaison rotule pour le pivotement de la tige de liaison. En effet, on remarque que cet assemblage ne permet pas un débattement suffisant de la tige de liaison lors du montage de l'implant rachidien, car ladite tige vient buter contre les bords extérieurs supérieur et inférieur de l'alésage.

C'est à ces inconvénients qu'entend plus particulièrement remédier la présente invention.

En effet le connecteur polyaxial pour implant rachidien suivant la présente invention à pour objet d'une part de pouvoir pivoter dans toutes les directions par rapport aux corps vertébraux et d'autre part de permettre un pivotement de grande amplitude de la tige de liaison par rapport audit connecteur.

Le connecteur polyaxial suivant la présente invention comprend :
- une tige de liaison, une vis de fixation comportant une première partie filetée pour son ancrage dans le tissu osseux, une tête intermédiaire à profil hexagonal et une seconde partie filetée recevant un écrou de serrage ;
- un premier élément de connexion percé d'un premier alésage prévu pour recevoir la seconde partie filetée de la vis de fixation, d'un second alésage comportant dans sa partie interne une piste annulaire à profil sphérique et d'une fente traversant le premier alésage pour venir déboucher à l'intérieur du second alésage au niveau de la piste annulaire ;
- un second élément de connexion percé d'un alésage prévu pour recevoir la tige de liaison, d'un trou fileté coopérant avec une vis de serrage pour le blocage en translation de ladite tige ;
- et un dispositif de liaison formant rotule qui permet, d'une part l'accouplement des premier et second éléments de connexion entre eux, de manière que lesdits éléments puissent pivoter l'un par rapport à l'autre pour présenter la tige de liaison dans une position angulaire déterminée, et d'autre part de décaler latéralement la tige de liaison par rapport au centre de pivotement desdits éléments.

Le connecteur polyaxial suivant la présente invention comporte un second élément de connexion pourvu du dispositif de liaison qui est constitué sur l'une des faces extérieures dudit élément d'un doigt qui se prolonge par une tête à profil sphérique de sorte que ladite tête puisse coopérer avec la piste annulaire à profil sphérique du second alésage du premier élément de connexion.

Le connecteur polyaxial suivant la présente invention comprend un premier élément dont le premier alésage comporte des portées cylindrique sur lesquelles viennent prendre appui respectivement l'écrou de serrage et la tête intermédiaire de la vis de fixation lors du blocage en rotation du dispositif de liaison par le serrage dudit écrou.

Le connecteur polyaxial suivant la présente invention comprend un premier élément de connexion qui présente de part et d'autre de la fente des branches réunies entre elles par une paroi courbe, lesdites branches étant susceptibles de se déformer sous l'effort de serrage de l'écrou de la vis de fixation pour le blocage en rotation des moyens de liaison.

Le connecteur polyaxial suivant la présente invention comprend un premier élément de connexion dont la fente est disposée dans un plan qui coupe perpendiculairement les axes principaux des alésages.

Le connecteur polyaxial suivant la présente invention comprend un second élément de connexion dont le trou fileté débouche à l'intérieur de l'alésage suivant une direction perpendiculaire.

La description qui va suivre en regard des dessins annexés, donnés à titre d'exemple non limitatif, permettra de mieux comprendre l'invention, les caractéristiques qu'elle présente et les avantages qu'elle est susceptible de procurer :
Figure 1 est une vue en perspective éclatée illustrant le connecteur polyaxial pour implant rachidien suivant la présente invention.
Figure 2 est une vue en perspective éclatée montrant en détail les premier et second éléments de connexion du connecteur polyaxial pour implant rachidien suivant la présente invention.
Figure 3 est une vue représentant les connecteurs polyaxiaux d'un implant rachidien fixés sur les vertèbres d'une colonne vertébrale.

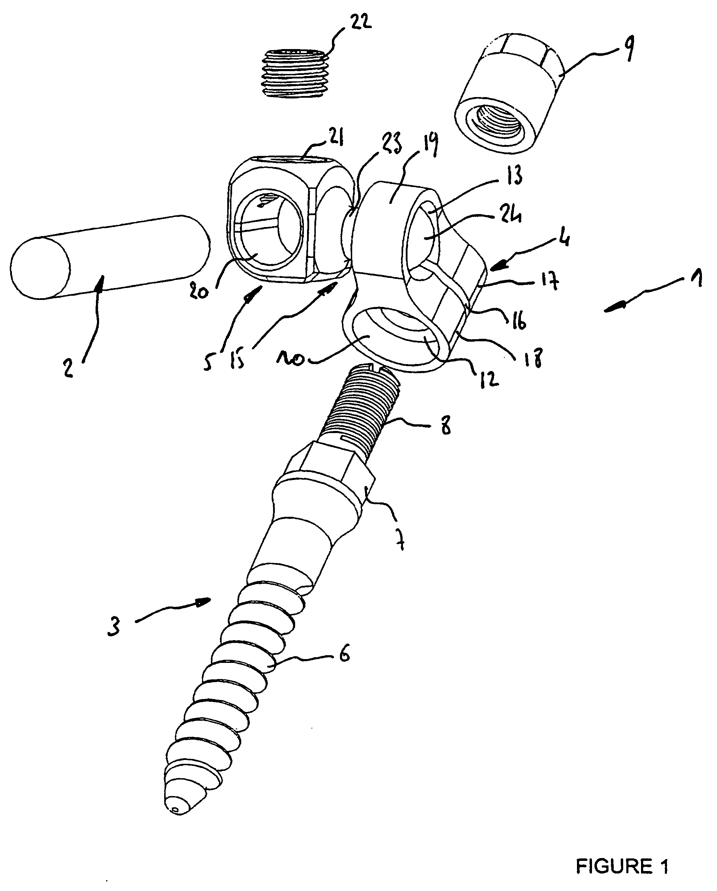

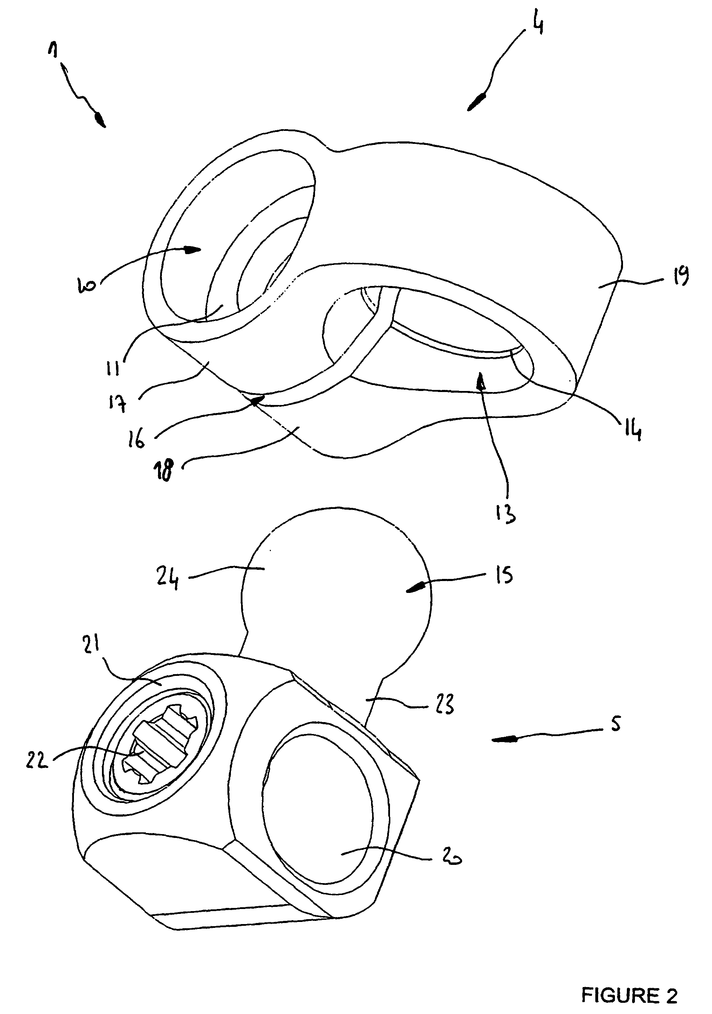

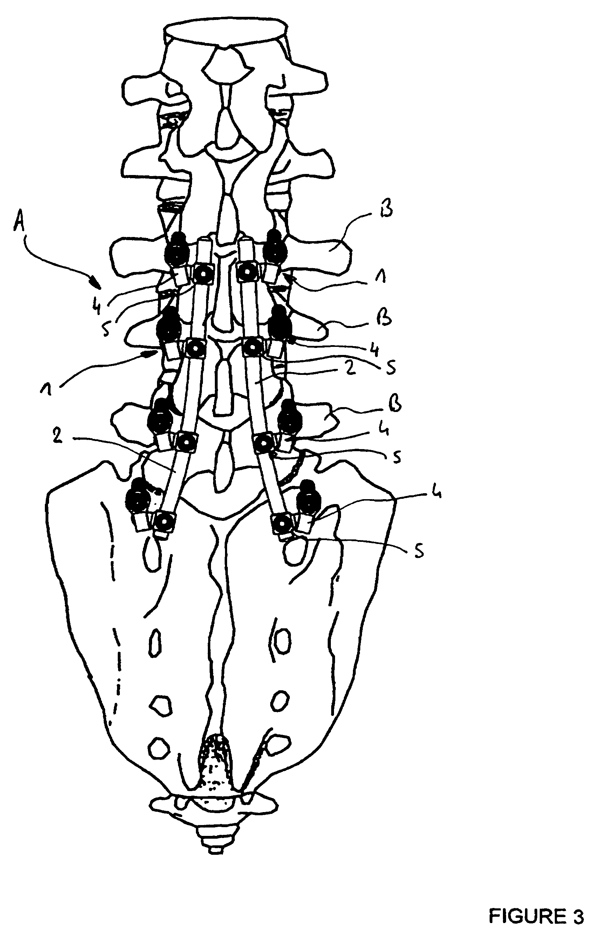

On a montré en figures 1 à 3 un connecteur polyaxial 1 permettant avec d'autres connecteurs de même genre la fixation d'une tige de liaison 2 pour la réalisation d'un implant rachidien A. Chaque connecteur polyaxial 1 de l'implant rachidien A est fixé sur le corps d'une vertèbre B d'une colonne vertébrale.

Ainsi, chaque connecteur polyaxial 1 de l'implant rachidien A comporte une vis de fixation 3, des premier et second éléments de connexion 4, 5 permettant la réception de la tige de liaison 2 et des moyens ou dispositif de liaison 15 formant rotule pour l'accouplement et le pivotement des éléments 4, 5 entre eux.

La vis de fixation 3 comporte une première partie filetée 6 pour son ancrage dans le tissu osseux d'une vertèbre B, une tête intermédiaire 7 à profil hexagonal formant butée, et une seconde partie filetée 8 s'étendant dans le prolongement et au-dessus de ladite butée.

La tête intermédiaire 7 permet l'entraînement en rotation de la vis de fixation 3 pour que la partie filetée 6 pénètre dans le tissu osseux de la vertèbre B correspondante.

La seconde partie filetée 8 est prévue pour recevoir un écrou 9 pour la fixation sur la vis 3 du premier élément de connexion 4 qui vient en appui contre la tête intermédiaire 7.

Le premier élément de connexion 4 est percé d'un alésage débouchant 10 comportant des portées cylindriques 11, 12 sur lesquelles viennent prendre appui respectivement l'écrou de serrage 9 et la tête intermédiaire 7 de la vis de fixation 3 lorsque celle-ci vient traverser l'alésage 10.

L'élément de connexion 4 est percé perpendiculairement à l'alésage 10 d'un autre alésage débouchant 13 qui comporte dans sa partie interne et en son milieu une piste annulaire 14 dont le profil est en portion de sphère.

L'élément de connexion 4 comporte une fente 16 traversant l'alésage 10 et débouchant dans l'alésage 13 au niveau de la piste annulaire 14. Ainsi, l'élément de connexion 4 présente de part et d'autre de la fente 16 des branches 17, 18 réunies entre elles par une paroi courbe 19 délimitant l'alésage 13. Les branches 17, 18 sont bien évidemment traversées chacune par l'alésage 10 pour le passage de la vis de fixation 3.

La fente 16 est disposée dans un plan qui coupe perpendiculairement les axes principaux des alésages débouchant 10 et 13.

La fente 16 permet de donner à l'élément de connexion 4 une certaine souplesse pour que celui-ci puisse se déformer sous l'effort de serrage de l'écrou 9 de la vis de fixation 3, afin que ledit élément de connexion 4 constitue une pince permettant le blocage angulaire des moyens de liaison 15 solidaire de l'élément de connexion 5 comme on le verra mieux plus loin.

L'élément de connexion 5 du connecteur polyaxial 1 est percé d'un alésage débouchant 20 destiné à recevoir la tige de liaison 2 de l'implant rachidien A.

L'élément de connexion 5 est percé perpendiculairement à l'alésage 20 d'un trou fileté 21 qui débouche à l'intérieur dudit alésage et qui est prévu pour recevoir une vis de serrage 22 pour le blocage en translation de la tige de liaison 2 par rapport à l'élément de connexion.

L'élément de connexion 5 comporte des moyens de liaison 15 qui sont constitués sur l'une des faces extérieures dudit élément d'un doigt 23 qui se prolonge par une tête 24 à profil sphérique.

Le premier élément de connexion 4 est retenu contre une vertèbre B d'un patient par l'intermédiaire de la vis de fixation 3 qui traverse l'alésage 10 prévu à cet effet.

Le premier élément de connexion 4 reçoit, avant le serrage de l'écrou 9 sur la seconde partie filetée 8 de la vis de fixation 3, le second élément de connexion 5 par l'intermédiaire des moyens de liaison 15 constitués de la tête sphérique 24 qui coopère avec la piste à profil sphérique 14 ménagée à l'intérieur de l'alésage 13 dudit premier élément de connexion.

Les moyens de fixation 15 permettent, d'une part d'accoupler ensemble les deux éléments de connexion 4, 5 du connecteur polyaxial 1, et d'autre part de réaliser entre les deux éléments de connexion une liaison rotule pour le réglage angulaire de la tige de liaison 2.

Les moyens de liaison 15 permettent également de décaler latéralement la tige de liaison 2 par rapport au centre de pivotement des éléments de connexion 4, 5 afin que ladite tige puissent présenter un débattement angulaire important.

La tige de liaison 2 est introduite dans l'alésage 20 de l'élément de connexion 5 et retenue transversalement à l'intérieur de celui-ci par l'intermédiaire de la vis de serrage 22 qui est vissée dans le trou fileté 21.

Lorsque la position angulaire de la tige de liaison 2 est déterminée par la rotation de la tête sphérique 24 à l'intérieur de la piste sphérique 14 de l'alésage 13, l'écrou 9 est serré sur la tête filetée 8 de la vis de fixation 3 de manière à déformer le premier élément de connexion 4 au moyen de la fente 16 afin de bloquer la tête sphérique 24 à l'intérieur de la piste 14.

Lors du blocage des moyens de liaison 15, on remarque que la butée 7 et l'écrou 9 de la vis de fixation 3 viennent respectivement en appui contre la portée 11 et 12 de l'alésage 10.

On note lors du serrage de l'écrou 9 de la vis de fixation 3 que les éléments de connexions 4 et 5 ne viennent pas en contact avec l'articulation de la vertèbre B correspondante du fait de position de la butée 7 sur ladite vis.

## Revendications

1. Connecteur polyaxial pour la réalisation d'un implant rachidien (A) afin de maintenir suivant des positions angulaires déterminées une tige de liaison (2) par rapport aux corps vertébraux (B), ledit connecteur (1) comprenant des alésages dont un est prévu pour recevoir la tige de liaison (2) et un deuxième pour coopérer avec une vis de fixation (3) comportant une première partie filetée (6) pour son ancrage dans le tissu osseux, une tête intermédiaire (7) à profil hexagonal et une seconde partie filetée (8) recevant un écrou de serrage (9), **caractérisé en ce que** chaque connecteur polyaxial (1) comprend :
• une vis de fixation (3) constituée d'une première partie filetée (6), d'une tête intermédiaire (7) à profil hexagonal formant butée et d'une second partie filetée (8) recevant un écrou de serrage (9) ;
• un premier élément de connexion (4) percé d'un premier alésage (10) prévu pour recevoir la seconde partie filetée (8) de la vis de fixation (3), d'un second alésage (13) comportant dans sa partie interne une piste annulaire (14) à profil sphérique, et d'une fente (16) traversant le premier alésage (10) pour venir déboucher à l'intérieur du second l'alésage (13) au niveau de la piste annulaire (14) ;
• un second élément de connexion (5) percé d'un alésage (20) prévu pour recevoir la tige de liaison (2), d'un trou fileté (21) coopérant avec une vis de serrage (22) pour le blocage en translation de ladite tige ;
• et un dispositif de liaison (15), formant rotule, qui permet, d'une part l'accouplement des premier et second éléments de connexion (4, 5) entre eux de manière que lesdits éléments puissent pivoter l'un par rapport à l'autre pour présenter la tige de liaison (2) dans des positions angulaires déterminées, et d'autre part de décaler latéralement la tige de liaison (2) par rapport au centre de pivotement desdits éléments (4, 5).

2. Connecteur polyaxial suivant la revendication 1, **caractérisé en ce que** le second élément de connexion (5) comporte un dispositif de liaison (15) qui est constitué sur l'une des faces extérieures dudit élément d'un doigt (23) qui se prolonge par une tête (24) à profil sphérique de sorte que ladite tête puisse coopérer avec la piste annulaire (14) à profil sphérique du second alésage (13) du premier élément de connexion (4).

3. Connecteur polyaxial suivant la revendication 1, **caractérisé en ce que** le premier alésage (10) comporte des portées cylindriques (11, 12) sur lesquelles viennent prendre appui respectivement l'écrou de serrage (9) et la tête intermédiaire (7) de la vis de fixation (3) lors du blocage en rotation du dispositif de liaison (15) par le serrage dudit écrou (9).

4. Connecteur polyaxial suivant la revendication 1, **caractérisé en ce que** le premier élément de connexion (4) présente de part et d'autre de la fente (16) des branches (17, 18) réunies entre elles par une paroi courbe (19), lesdites branches étant susceptibles de se déformer sous l'effort de serrage de l'écrou (9) de la vis de fixation (3) pour le blocage en rotation du dispositif de liaison (15).

5. Connecteur polyaxial suivant la revendication 1, **caractérisé en ce que** la fente (16) est disposée dans un plan qui coupe perpendiculairement les axes principaux des alésages (10, 13) du premier élément de connexion (4).

6. Connecteur polyaxial suivant la revendication 1, **caractérisé en ce que** le second élément de connexion (5) comporte un trou fileté (21) qui débouche suivant une direction perpendiculaire à l'intérieur de l'alésage (20).

## Patentansprüche

1. Mehrachsiger Verbinder für die Herstellung eines Wirbelsäulenimplantats (A), um einen Verbindungsschaft (2) entlang bestimmten Winkelpositionen relativ zu den Wirbelkörpern (B) zu halten, wobei der Verbinder (1) Bohrungen umfasst, von denen eine dafür vorgesehen ist, den Verbindungsschaft (2) aufzunehmen, und eine zweite, um mit einer Befestigungsschraube (3) zusammenzuwirken, die einen ersten, mit einem Gewinde versehenen Teil (6) für ihre Verankerung in dem Knochengewebe, einen Zwischenkopf (7) mit hexagonalem Profil und einen zweiten, mit einem Gewinde versehenen Teil (8) umfasst, der eine Druckmutter (9) aufnimmt, **dadurch gekennzeichnet, dass** jeder mehrachsige Verbinder (1) Folgendes umfasst:
eine Befestigungsschraube (3), die aus einem ersten, mit einem Gewinde versehenen Teil (6), einem ein Widerlager bildenden Zwischenkopf (7) mit hexagonalem Profil und einem zweiten, mit einem Gewinde versehenen Teil (8) besteht, der eine Druckmutter (9) aufnimmt;
ein erstes Verbindungselement (4), das durchquert wird von einer ersten Bohrung (10), die dafür vorgesehen ist, den zweiten, mit einem Gewinde versehenen Teil (8) der Befestigungsschraube (3) aufzunehmen, einer zweiten Bohrung (13), die in ihrem inneren Teil eine ringförmige Bahn (14) mit kugelförmigem Profil aufweist, und einem Schlitz (16), der die erste Bohrung (10) durchquert, um im Inneren der zweiten Bohrung (13) auf Höhe der ringförmigen Bahn (14) zu münden;
ein zweites Verbindungselement (5), das durchquert wird von einer ersten Bohrung (20), die dafür vorgesehen ist, den Verbindungsschaft (2) aufzunehmen, und einer Gewindebohrung (21), die mit einer Druckschraube (22) für die Translationsblockade des Schafts zusammenwirkt;
und eine ein Kugelgelenk bildende Verbindungsvorrichtung (15), die einerseits die Kopplung des ersten und zweiten Verbindungselements (4, 5) untereinander gestattet, so dass die Elemente relativ zueinander schwenken können, um den Verbindungsschaft (2) in bestimmten Winkelpositionen zu präsentieren, und es andererseits gestattet, den Verbindungsschaft (2) relativ zum Schwenkzentrum der Elemente (4, 5) seitlich zu verschieben.

2. Mehrachsiger Verbinder nach Anspruch 1, **dadurch gekennzeichnet, dass** das zweite Verbindungselement (5) eine Verbindungsvorrichtung (15) umfasst, die auf einer der Außenflächen des Elements aus einem Finger (23) besteht, der sich in einem Kopf (24) mit kugelförmigem Profil fortsetzt, so dass der Kopf mit der ringförmigen Bahn (14) mit kugelförmigem Profil der zweiten Bohrung (13) des ersten Verbindungselements (4) zusammenwirken kann.

3. Mehrachsiger Verbinder nach Anspruch 1, **dadurch gekennzeichnet, dass** die erste Bohrung (10) zylindrische Auflager (11, 12) trägt, auf denen die Druckmutter (9) beziehungsweise der Zwischenkopf (7) der Befestigungsschraube (3) bei der Drehblockade der Verbindungsvorrichtung (15) durch das Anziehen der Mutter (9) zur Anlage kommen.

4. Mehrachsiger Verbinder nach Anspruch 1, **dadurch gekennzeichnet, dass** das erste Verbindungselement (4) auf beiden Seiten des Schlitzes (16) Arme (17, 18) aufweist, die untereinander durch eine gekrümmte Wandung (19) verbunden sind, wobei die Arme sich unter der Anzugskraft der Mutter (9) der Befestigungsschraube (3) für die Drehblockade der Verbindungsvorrichtung (15) deformieren können.

5. Mehrachsiger Verbinder nach Anspruch 1, **dadurch gekennzeichnet, dass** der Schlitz (16) in einer Ebene angeordnet ist, die die Hauptachsen der Bohrungen (10, 13) des ersten Verbindungselements (4) senkrecht schneidet.

6. Mehrachsiger Verbinder nach Anspruch 1, **dadurch gekennzeichnet, dass** das zweite Verbindungselement (5) eine Gewindebohrung (21) umfasst, die in einer Richtung senkrecht zum Inneren der Bohrung (20) mündet.

## Claims

1. Multiaxis connector for producing a spinal implant (A) to keep a connecting rod (2) in determined angular positions with respect to the vertebral bodies (B), said connector (1) comprising bores, one of which is intended to receive the connecting rod (2), and a second of which is intended to cooperate with a fixing screw (3) comprising a first threaded part (6) for anchoring it into the bone tissue, an intermediate head (7) of hexagonal profile and a second threaded part (8) that takes a clamping nut (9), **characterized in that** each multiaxis connector (1) comprises:
• a fixing screw (3) consisting of a first threaded part (6), an intermediate head (7) with a hexagonal profile forming a stop, and a second threaded part (8) receiving a clamping nut (9);
• a first connecting element (4) pierced with a first bore (10) designed to receive the second threaded part (8) of the fixing screw (3), with a second bore (13), comprising, in its interior part, an annular track (14) of spherical profile, and with a slot (16) passing through the first bore (10) to open into the second bore (13) at the annular track (14);
• a second connecting element (5) pierced with a bore (20) designed to receive the connecting rod (2), with a threaded hole (21) collaborating with a binding screw (22) for immobilizing said rod in terms of translation;
• and a connecting device (15) forming a ball joint which, on the one hand, allows the first and second connecting elements (4, 5) to be coupled to one another in such a way that said elements can pivot one with respect to the other so as to present the connecting rod (2) in determined angular positions and, on the other hand, allow the connecting rod (2) to be offset laterally with respect to the centre of pivoting of said elements (4, 5).

2. Multiaxis connector according to Claim 1, **characterized in that** the second connecting element (5) comprises a connecting device (15) which consists, on one of the exterior faces of said element, of a finger (23) extended by a head (24) with a spherical profile so that said head can collaborate with the annular track (14) of spherical profile of the second bore (13) of the first connecting element (4).

3. Multiaxis connector according to Claim 1, **characterized in that** the first bore (10) comprises cylindrical bearing surfaces (11, 12) on which the clamping nut (9) and the intermediate head (7) of the fixing screw (3) respectively bear when the connecting device (15) is immobilized in terms of rotation by the tightening of said nut (9).

4. Multiaxis connector according to Claim 1, **characterized in that** the first connecting element (4) has, on each side of the slot (16), branches (17, 18) joined together by a curved wall (19), said branches being able to deform under the clamping force of the nut (9) of the fixing screw (3) to immobilize the connecting device (15) in terms of rotation.

5. Multiaxis connector according to Claim 1, **characterized in that** the slot (16) is arranged in a plane which intersects the main axes of the bores (10, 13) of the first connecting element (4) at right angles.

6. Multiaxis connector according to Claim 1, **characterized in that** the second connecting element (5) comprises a threaded hole (21) which opens into the bore (20) in a perpendicular direction.
